# EUROPEAN PATENT APPLICATION

(11) **EP 4 219 674 A1**
(43) Date of publication of application: **02.08.2023**
(21) Application number: 23152028.9
(22) Date of filing: 17.01.2023
(51) Int. Cl.: C12M 1/00, C12M 1/26, C12M 1/36

(54) **IN-TIME STORAGE OF MANUFACTURED CELLS FOR MEDICAL CELLULAR THERAPY GROWN AND DIFFERENTIATED IN BIOREACTORS**

(30) Priority: 31.01.2022 US 202263304934 P
(71) Applicant: Sciperio, Inc., Orlando, Florida 32826 (US)
(72) Inventor: MOSER, Janice M., Oviedo, 32766 (US); PERKOWSKI, Casey W., Winter Park, 32792 (US); BUSSE, Pierce J., Pensacola, 32503 (US); HAUPFEAR, Kelly, Orlando, 32826 (US); CHURCH, Kenneth H., Orlando, 32828 (US)
(74) Representative: Boult Wade Tennant LLP

(57) **Abstract**

A system includes a bioreactor having an inlet and an outlet, a cell-media recirculation loop between the outlet of the bioreactor and the inlet of the bioreactor, and a mature cell separation unit positioned along the cell-media recirculation loop wherein the mature cell separation unit is configured for performing separation of mature cells from immature cells and direction of the immature cells back to the inlet of the bioreactor. The system may further include a storage unit for storing the mature cells. The mature cell separation unit may be further configured to direct the mature cells to a mature cell storage unit.

## Description

### RELATED APPLICATIONS

This application claims priority to U.S. Provisional Patent Application No. 63/304,934 filed January 31, 2022, hereby incorporated by reference in its entirety.

### FIELD OF THE INVENTION

The present invention relates to bio processing or cell cultures. More particularly, but not exclusively, the present invention relates to apparatuses, methods, and systems for autonomous separation and/or storage of manufactured cells.

### BACKGROUND OF THE INVENTION

Cell culture growth for both cell-based therapies as well as biologics production typically use bioreactors for product manufacturing. In the cases where the biologic product is the cells themselves, typically the cell culture is extracted wholly at the end of the cell growth protocol. Current technology may use filters to isolate desired cellular product from waste, however, such methods may result in a high rate of production loss. Moreover, filtering final product from waste only at the end of a cell growth protocol may reduce the Bioreactor's overall efficiency, as cell cultures tend to have a window during growth and maturation where a given cell is viable and acceptable for a final product. This reduction in efficiency can be the difference in making a cell-based product economically viable for cell cultures in which mature cell viability has a narrow stability window. Therefore, problems remain associated with cell culture growth.

### SUMMARY

Therefore, it is a primary object, feature, or advantage of the present invention to improve over the state of the art.

It is another object, feature, or advantage to increase efficiency of cell culture based production.

It is a further object, feature, or advantage to provide for extracting mature viable cells from a cell culture during the process of the cell growth protocol.

Yet a further object, feature, or advantage is to make cell-based products more economically viable.

One or more of these and/or other objects, features, or advantages will become apparent from the specification and claims that follow. Different embodiments may have different objects, features, or advantages. Thus, not every embodiment will have each or every object, features, or advantages and the present invention should be limited by or to these objects, features, or advantages.

According to another aspect, a system includes a bioreactor having an inlet and an outlet, a cell-media recirculation loop between the outlet of the bioreactor and the inlet of the bioreactor, and a mature cell separation unit positioned along the cell-media recirculation loop wherein the mature cell separation unit is configured for performing separation of mature cells from immature cells and direction of the immature cells back to the inlet of the bioreactor. The system may further include a storage unit for storing the mature cells. The mature cell separation unit may be further configured to direct the mature cells to a mature cell storage unit. The mature cells are mature red blood cells. The mature cell separation unit includes at least one sensor and at least one intelligent control operatively connected to the at least one sensor for monitoring the separation of mature cells from the immature cells. The mature cells may be mature human cells such as mature red blood cells. The at least one intelligent control provides for controlling the separation of the mature cells from the immature cells. The at least one intelligent control provides for controlling the direction of the mature cells from the immature cells. The mature cell separation unit may be further configured to direct the mature cells to a mature cell storage unit and the at least one intelligent control may provide for controlling direction of the mature cells to the mature cell storage unit. The mature cell separation unit may be further configured to direct the mature cells to a mature cell storage unit using at least one of filters, cyclonic separation, and microfluidics. The storage unit may be a temperature controlled storage unit.

According to another aspect, a method for autonomous segregation of mature cells from a culture includes autonomously monitoring cell-media in a culture within a cell-media recirculation loop associated with a bioreactor and autonomously separating the mature cells from the culture within the cell media re-circulation loop. The method may further include storing the mature cells after autonomously separating the mature cells from the culture. The step of autonomously monitoring of the cell media within the cell media re-circulation loop may be performed using a control system comprising at least one sensor operatively connected to an intelligent control. The mature cell separation unit may be further configured to direct the mature cells to a mature cell storage unit using at least one of filters, cyclonic separation, and microfluidics.

According to another aspect, a method for autonomous extraction of mature cells from a cell culture is provided. The method may include monitoring cell-media within a cell culture and autonomously extracting the mature cells from the cell culture. The cell-media may be within a re-circulation loop associated with a bioreactor. The monitoring of the cell media may be performed using environmental sensors. The environmental sensors may include at least one of pH sensors, and dissolved oxygen sensors.

According to yet another aspect, the present invention relates to process and hardware for autonomous separation of cells following non-uniform expansion and differentiation in bioreactors to protect them from negative effects of being continuously cultured in a 37 °C bioreactor following maturation. This in-process separation would benefit cultures such as differentiated, enucleated red bloods cells (RBCs) from a bioreactor stir-based bioreactor to store at 4°C.

### BRIEF DESCRIPTION OF THE DRAWINGS

FIG. 1 is a block diagram illustrating one overview of a system.
FIG. 2 is a block diagram illustrating one example of a control system.

### DETAILED DESCRIPTION

It has been discovered by the present inventors that a percentage of manufactured RBCs (mRBCs) (red blood cells which are grown in culture from a stem cell or progenitor cell) exhibit storage lesions when analyzed at the end of the culturing process. Although the mRBCs exhibit all the traditional surface markers associated with red blood cells, several of the cells demonstrate a morphological shape (spheroechinocyte) that is not the traditional concave disk or doughnut shape of an RBC. They exhibit spikes on their surface which have been documented in donated RBCs after they have been stored for extended time and as they approach expiration. The morphological, oxidative, and metabolic changes compromise the effectiveness of oxygen transport posttransfusion, cell viability, and ability to be stored. The present inventors believe the lesions are due to retention of the cells at traditional cell culture parameters, such as 37°C temperature with mechanical agitation, that are no longer optimal for mature RBCs and by pulling out the mature mRBCs which have enucleated and have not been exposed to extended post-enucleation culturing, they will not develop cellular damage and lesions. In the research setting, high product loss can be a non-issue, however, commercially the high product loss rate is a significant contributing factor for lack of economical viability for manufactured RBCs.

Therefore, apparatus, methods, and systems are disclosed herein for recirculation of media during a biological process so that mature cells may be separated while the process is continuing to mature additional cells.

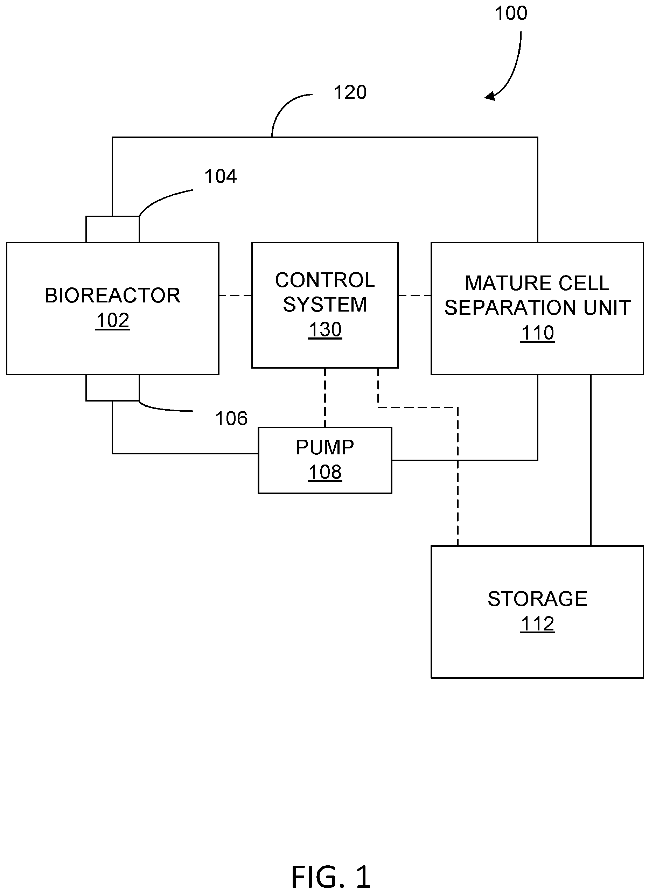

FIG. 1 is a diagram of one example of a system 100. The system 100 includes a bioreactor 102. The bioreactor 102 has an inlet 104 and an outlet 106. The pump 108 is used to circulate fluid in re-circulation loop 120. A mature cell separation unit 110 is also within the re-circulation loop 120. In operation, cell media is recirculated from the outlet 106 of the bioreactor 102 to the mature cell separation unit 110. At the mature cell separation unit 110, mature cells may be separated from the fluid and conveyed to a storage unit 112 while fluid containing cells which are not yet matured may re-circulate to the inlet 104 of the bioreactor.

The re-circulation loop 120 for the cell-media is external to the bioreactor 120. The cell-media may be circulated through the re-circulation loop 120 during operation of the bioreactor 102. The re-circulation loop provides an additional flow path such as a tube, channel, pipe, or other conduit for the cell culture media solution. The cell culture media solution contained within the re-circulation loop 120 is driven by the pump 108. The pump 108 may be a peristaltic pump, a syringe, an impeller, or other type of pump suitable for conveying the cell culture media solution through the re-circulation loop 120. In operation, a portion or percentage of the solution volume from within the bioreactor 102 is transported to a location external from the bioreactor volume before the solution is returned to back to the bioreactor 102. The process does not change or does not significantly change the bioreactor volume.

The mature cell separation unit provides for separating enucleated from nucleated cells Although various types of cell separation are known to one skilled in the art, here, the mature cell separation unit is positioned in-line is not novel and has been documented. The use of this device in line with a bioreactor and for use in pulling enucleated cells from culture before during an active cell culture growth is highly novel. The mature cell separation unit 110 may be based on any cell separation method and implemented in a continuous or intermittent manner. Traditionally continuous separation would be achieved with filtration methods, such as Tangential Flow Filtration (TFF) or basic filtering using size elimination filters. However, these devices are troublesome when used in-line with a bioreactor, mainly due to clogging and loss separation effectiveness overtime. Microfluidics-based separation devices may be used. These devices use complex fluid-dynamic effects that occur on the micrometer scale. Common passive techniques proven effective in the lab include but are not limited to, Inertial Particle Lift (IPL), Deterministic Lateral Displacement (DLD), and Pinched Flow Fractionation (PFF). Active Separation techniques include the use of electro -magnetic fields, acoustical waves, and laser to accurately separate the cell culture at a cell by cell basis. Microfluidics although very efficient, and not prone to clogging (this is dependent on channel geometry) are not without flaws, efficient devices are highly dependent on channel geometry, flow rate, and cell properties and must be optimized to the cell type of interest. Moreover, volume flow rates through micro channels are often low, requiring a large number of channels to process high volumes. Other separation methods such as centrifugation can also be used, in an intermittent manor. Thus, it is contemplated that numerous types of cell separation units may be used.

The storage unit 112 allows for holding or storing the enucleated mRBCs (or other type of target cells). The storage unit 112 may be temperature-controlled across a broad range of temperatures. This maybe be a simple test tube which is collected in a certain time interval and moved to storage by a technical or a more advanced chilled storage container, which may be collected as a whole at the end of a cell culture run. The storage unit 112 may have various sensors, including, pH and dissolved oxygen sensors with the ability to cool the container to near 4°C and introduce gaseous nitrogen to create a hypoxic environment and carbon dioxide to control pH levels. The addition of nitrogen to generate a hypoxic environment will slow the rate of glycolysis in the cells and there prevent oxidative and metabolic damage typically seen with all stored RBCs post-donation or manufacture.

A control system 130 is shown which may be electrically connected to otherwise in operative communication with the bioreactor 102, the pump 108, the mature cell separation unit 110, and the storage unit 112.

The control system 130 allows for autonomous operation. For example, once the system is set-up for the cell-culture, the system need not require any input from a user until after completion of the cell culture protocol. Thus, some level of automation data measurement and control is required to allow for autonomous operation. There may be sensors measuring cell culture and flow data. A computing device or other intelligent control may read the data and make control decisions in order to optimize cell separation. For example, flow rate though the microfluidic chip can be measured and adjusted to increase cell separation efficiency.

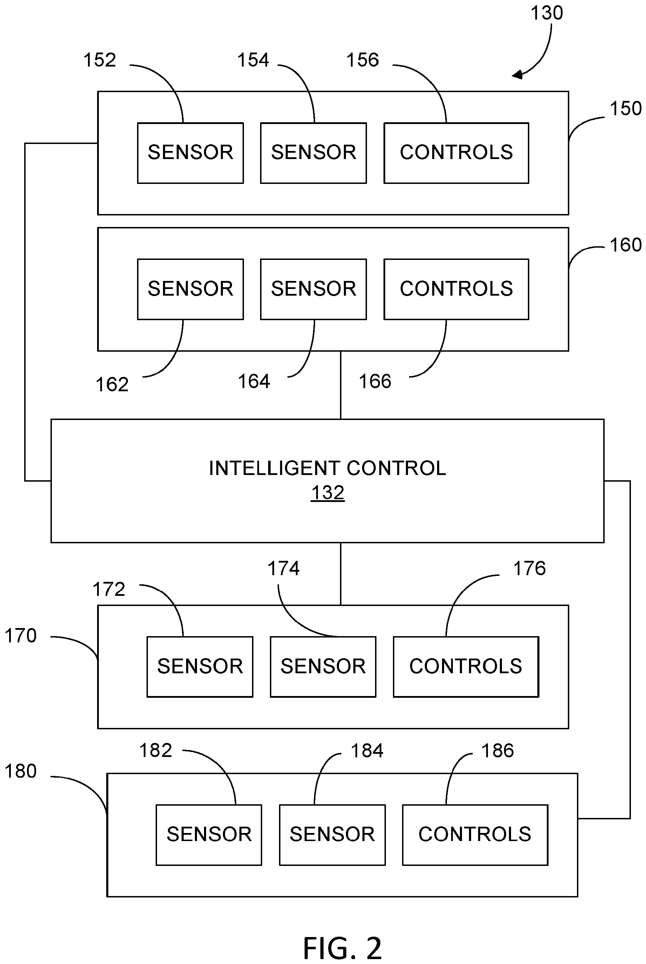

FIG. 2 is a diagram illustrating one example of a control system 130 in more detail. As shown in FIG. 2, the control system 130 may include an intelligent control 132 and may be connected to one or more sensors or controls including sensors and/or controls associated with the bioreactor, mature cell separation unit, storage unit, and pump. The control system 130 may include an intelligent control such as a processor, microcontroller, logic circuit, or other type of intelligent control. In some embodiments, each of the bioreactor, mature cell separation unit, storage unit, and pump may have its own control system and may communicate with the intelligent control 132 over a network such as I2C, SPI, serial network, ethernet network, CAN, Bluetooth, or other type of wired or wireless network. In such embodiments monitoring and control functions may be distributed between various components of the system.

A first set of sensors and/or controls 150 may be associated with the bioreactor. A second set of sensors and/or controls 160 may be associated with the mature cell separation unit. A third set of sensors and/or controls 170 may be associated with the storage unit. A fourth set of sensors and/or controls 180 may be associated with the pump. Various types of sensors and controls may be present based on the function(s) to be performed.

For example, the first set of sensors and/or controls 150 may include one or more sensors which monitors progress of a biological process performed in the bioreactor. Sensors 152, 154 may be used to determine that a portion of cells within media within the bioreactor have matured or have likely matured. Examples of such sensors may include pH sensors, ORP/redox sensors, dissolved CO2 sensors, dissolved oxygen sensors, gas sensors, temperature sensors, or other types of sensors. The controls 156 may include actuators controls or other types of controls. For example, the controls 156 may include actuator controls for opening and/or closing the inlet and outlets to the bioreactor.

The second set of sensors and/or controls 160 associated with the mature cell separation unit may include sensors used in the process of identifying and/or extracting mature cells from the cell culture. This separation process may be achieved in any number of different ways including those previously discussed. The type of cell separation used may be selected based on the type of cells, the required volume or speed, and other considerations. Thus sensors 162, 164 may include sensors associated with the separation process such as sensors for measuring flow rates, and controls 166 may be associated with controls associated with the separation process including actuators, switches or otherwise.

The third set of sensors and/or controls 170 may be associated with the storage unit. The storage unit may be refrigerated. The sensors 172, 174 may include temperature sensors, sensors for determining amount or volume of cells being stored or remaining capacity of the storage unit. The controls 176 may be used to switch a cooling unit on, open or close valves, or otherwise control the storage unit. This third set of sensors and/or controls 170 may be used to set and maintain optimum or otherwise desirable storage environment conditions.

Although, the apparatus, systems, and methods described herein have generally been in the context of red blood cell bioreactors, one skilled in the art would recognize that the present disclosure supports application to other types of cell culture products. For example, other types of cell culture products where the cell culture product reaches maturity non-uniformly, especially where this lack of absolute process uniformity during differentiation results in negative biological effects on the end-product, there are no non-trivial or no method(s) of forcing cell differentiation/maturity uniformity, and long term storage conditions vary greatly from the conditions present inside of the Bioreactor environment.

As such, it follows that the invention specialized for the RBC maturity problem, can be generalized to all cells which would benefit from removing mature cells during the cell culture protocol such as cells developed for any type of immunotherapy. Additional examples include CAR T-cells to treat cancer, skin cells for wound or burn repair, and beta cells to treat diabetes.

The present invention contemplates numerous options, variations, and alternatives. This includes variations in the type of cell culture product, the type of bioreactor(s), the structure of the cell-media recirculation loop, the type of mature cell separation unit, the type of storage unit, the type of control system, the selection of sensors, and controls, and other variations. The present invention is not to be limited to the specific embodiments shown or described herein.

## Claims

1. A system comprising:
a bioreactor having an inlet and an outlet;
a cell-media recirculation loop between the outlet of the bioreactor and the inlet of the bioreactor; and
a mature cell separation unit positioned along the cell-media recirculation loop wherein the mature cell separation unit is configured for performing separation of mature cells from immature cells and direction of the immature cells back to the inlet of the bioreactor.

2. The system of claim 1 further comprising a storage unit for storing the mature cells.

3. The system of claim 2 wherein the storage unit is a temperature-controlled storage unit.

4. The system of claim 3 wherein the mature cell separation unit is further configured to direct the mature cells to a mature cell storage unit using at least one of filters, cyclonic separation, and microfluidics.

5. The system of claim 1 wherein the mature cells are mature red blood cells.

6. The system of claim 1 wherein the system comprises at least one sensor and at least one intelligent control operatively connected to the at least one sensor.

7. The system of claim 6 wherein the mature cells are mature red blood cells.

8. The system of claim 6 wherein the at least one intelligent control provides for controlling the separation of the mature cells from the immature cells.

9. The system of claim 8 wherein the at least one intelligent control provides for controlling the direction of the mature cells from the immature cells.

10. The system of claim 6 wherein the mature cell separation unit is further configured to direct the mature cells to a mature cell storage unit and wherein the at least one intelligent control provides for controlling direction of the mature cells to the mature cell storage unit.

11. The system of claim 10 wherein the mature cells are mature human cells.

12. The system of claim 10 wherein the mature cells are mature red blood cells.

13. A method for autonomous segregation of mature cells from a culture, the method comprising:
autonomously monitoring cell media in the culture within a cell media re-circulation loop associated with a bioreactor; and
autonomously separating the mature cells from the culture within the cell media recirculation loop.

14. The method of claim 13 further comprising storing the mature cells after autonomously separating the mature cells from the culture.

15. The method of claim 13 wherein the autonomously monitoring of the cell media within the cell media re-circulation loop is performed using a control system comprising at least one sensor operatively connected to an intelligent control.
